# EUROPÄISCHE PATENTSCHRIFT

(11) **EP 1 332 110 B1**
(45) Veröffentlichungstag und Bekanntmachung des Hinweises auf die Patenterteilung: **25.07.2007**
(21) Anmeldenummer: 01982456.4
(22) Anmeldetag: 02.11.2001
(51) Int. Cl.: C01C 1/02, C07D 251/60

(54) **VERFAHREN ZUR REINIGUNG VON MELAMINHÄLTIGEM AMMONIAK**
METHOD FOR THE PURIFICATION OF MELAMINE-CONTAINING AMMONIA
PROCEDE D'EPURATION D'AMMONIAC RENFERMANT DE LA MELAMINE

(30) Priorität: 08.11.2000 AT 18882000
(43) Veröffentlichungstag der Anmeldung: 06.08.2003
(73) Patentinhaber: AMI Agrolinz Melamine International GmbH, 4020 Linz (AT)
(72) Erfinder: COUFAL, Gerhard, A-4060 Leonding (AT)
(74) Vertreter: Gross, Felix
(86) Internationale Anmeldenummer: PCT/EP2001/012691
(87) Internationale Veröffentlichungsnummer: WO 2002/038498

(56) Entgegenhaltungen:
- WO-A-00/39107
- WO-A-97/20826
- US-A- 3 555 784
- US-A- 4 251 235
- US-A- 4 565 867
- US-A- 5 721 363

## Beschreibung

Die Anmeldung betrifft ein Verfahren zur Reinigung von gasförmigem melaminhältigem Ammoniak. Das erfindungsgemäße Verfahren ist insbesondere zur Reinigung von aus Melaminhochdruckanlagen mit wasserfreier Melaminaufarbeitung stammenden Ammoniakgasen geeignet.

Bei den Hochdruckverfahren zur Herstellung von Melamin wird Harnstoff in einer endothermen Flüssigphasenreaktion zu Melamin umgesetzt. Nach Abtrennen von NH₃ und CO₂ wird eine unter hohem Druck stehende Melaminschmelze erhalten, die anschließend in den sogenannten nassen Aufarbeitungsverfahren durch Quenchen mit Wasser verfestigt wird. Bei den sogenannten trockenen Verfahren, wie sie beispielsweise in US 4,565,867, WO 95/01345, WO 97/20826 oder WO99/38852 beschrieben sind, wird die Melaminschmelze durch Quenchen mit Ammoniak, durch Entspannen der mit NH₃ gesättigten Melaminschmelze oder einer Melamin/NH₃-Suspension bei einer Temperatur knapp oberhalb des Melaminschmelzpunktes, durch Sublimation mit nachfolgender Desublimation oder durch Abkühlen in einem Wirbelbett verfestigt. In diesen Verfahren fallen zum Teil sehr große Mengen an gasförmigem NH₃ an, die jedoch je nach Druck und Temperatur des anfallenden Gases mehr oder minder große Mengen an Melamin enthalten. Dieses, bereits CO₂-freie Gas kann nur teilweise und zum Teil nur mit Schwierigkeiten in die Melaminanlage rückgeführt werden. Das im NH₃-Gas enthaltene Melamin verursacht insbesondere in den Kompressoren Schwierigkeiten und Störungen, wenn das NH₃-Gas zur Verflüssigung komprimiert werden muß.

Es stellte sich demnach die Aufgabe, melaminhältiges NH₃-Gas in einem einfachen Verfahren von Melamin zu reinigen. Diese Aufgabe konnte mit Hilfe der Erfindung dadurch gelöst werden, daß das melaminhältige, gasförmige NH₃ teilweise kondensiert wird. Dabei wird einerseits melaminhältiges, flüssiges NH₃ und andererseits gereinigtes, melaminfreies NH₃-Gas erhalten, das vom flüssigen NH₃ abgetrennt wird.

Gegenstand der Erfindung ist demnach ein Verfahren zur Reinigung von melaminhältigem, gasförmigem NH₃, das dadurch gekennzeichnet ist, daß es durch Abkühlen teilweise kondensiert wird, wobei sich das Melamin im flüssigen NH₃ anreichert und das gereinigte NH₃-Gas abgezogen wird.

Das Abkühlen des melaminhältigen, gasförmigen NH₃ kann mittels irgendeiner Kühleinrichtung erfolgen, beispielsweise in einer Kühlkolonne oder mittels flüssigem NH₃, wobei flüssiges NH₃ mit dem zu reinigenden NH₃ vermischt wird, beispielsweise durch Einsprühen von flüssigem NH₃ in das zu reinigende NH₃ oder durch Durchleiten des zu reinigenden NH₃ durch flüssiges NH₃. Bevorzugt wird das melaminhältige gasförmige NH₃ durch flüssiges NH₃ geleitet, wobei es sich abkühlt und teilweise kondensiert. Das dabei erhaltene, gereinigte NH₃-Gas wird abgezogen und kann gegebenenfalls kondensiert werden, das mit Melamin angereicherte flüssige NH₃ wird ausgeschleust. Die Menge des ausgeschleusten flüssigen NH₃ wird bevorzugt durch das kondensierende NH₃-Gas ersetzt, es kann jedoch auch durch Zufuhr von frischem NH₃ ersetzt werden.

Das zu reinigende, melaminhälte NH₃-Gas ist bereits CO₂-frei und stammt bevorzugt aus trockenen Melaminverfahren, wie oben angeführt, etwa aus Verfahren zum Verfestigen von flüssigem oder gasförmigem, unter NH₃-Druck stehendem Melamin oder einer Melamin/NH₃-Suspension mit Hilfe von flüssigem, überkritischem oder gasförmigem NH₃. Die Verfestigung bei den trockenen Melaminverfahren erfolgt beispielsweise durch Quenchen mit NH₃, wobei NH₃ und flüssiges oder gasförmiges Melamin in einen Quencher gesprüht werden. Besonders bevorzugt stammt das zu reinigende, melaminhältige NH₃-Gas aus dem Wirbelbett einer Anlage zur Verfestigung von Melamin, in die das flüssige oder gasförmige Melamin in ein aus festem Melamin oder festen Inertstoffteilchen und festem Melamin aufgebautes und mit NH₃-Gas aufrechterhaltenes Wirbelbett eingebracht wird.

Bevorzugt wird das Verfahren kontinuierlich durchgeführt. Das gasförmige, melaminhältige NH₃ wird dabei beispielsweise über einen mit Luft oder Wasser gekühlten Kühler oder durch direkten Kontakt mit flüssigem NH₃ teilweise kondensiert. Bevorzugt wird das gasförmige, melaminhältige NH₃ dabei durch flüssiges NH₃ geleitet, wobei sich Melamin im flüssigen NH₃ abscheidet bzw. löst und anreichert. Abhängig davon, aus welcher Anlage zur trockenen Melaminaufarbeitung das zu reinigende Gas kommt, kann es verschiedenen Druck und verschiedene Temperatur und in Abhängigkeit davon unterschiedlichen Melamingehalt aufweisen. Die Untergrenze der Temperatur liegt bevorzugt knapp über der jeweiligen Kondensationstemperatur beim jeweiligen Druck, die Obergrenze bei den Betriebstemperaturen des Anlagenteiles, aus dem das zu reinigende NH₃ stammt. Stammt das melaminhältige NH₃ beispielsweise aus einem Verfahren zur Verfestigung von gasförmigem Melamin mit Hilfe oder in Gegenwart von Ammoniak, so liegt beispielsweise der Druck bei etwa 1 - 20 bar, bzw. bei etwa 1,5 - 15 bar und die Temperatur beispielsweise bei etwa 290 - 520 °C. Stammt das melaminhältige NH₃ beispielsweise aus einem Verfahren zur Verfestigung von flüssigem Melamin, so sind auch niedrigere Temperaturen bis zu Raumtemperatur, bevorzugt von etwa 100 °C bis knapp unterhalb des druckabhängigen Schmelzpunktes des Melamins möglich, beispielsweise von etwa 100 bis 340 °C, besonders bevorzugt von etwa 200 bis 320 °C. Es sind auch höhere Drücke bis zu etwa 500 bar, bevorzugt von etwa 5 bis 250 bar, besonders bevorzugt von etwa 10 bis 100 bar möglich. Druck und Temperatur bei der teilweisen Kondensation müssen dabei so gewählt sein, daß mit dem zur Verfügung stehenden Kühlmittel das NH₃ kondensiert werden kann. Beispielsweise bei Verwendung des Kühlmittels Wasser soll der Druck entsprechend der Kondensationskurve von Ammoniak nicht unter 10 bar sein.

Das Verfahren läßt sich bevorzugt und besonders einfach derart durchführen, daß das melaminhältige, gasförmige NH₃ durch flüssiges NH₃ geleitet und teilweise kondensiert wird. Das dabei gereinigte NH₃-Gas kann dann abgezogen und das mit Melamin angereicherte flüssige NH₃ ausgeschleust werden. Die Menge des ausgeschleusten melaminhältigen flüssigen NH₃ wird dabei laufend durch kondensierendes NH₃ ersetzt. Es ist aber auch möglich, teilweise frisches flüssiges NH₃ zuzuführen. Die teilweise Kondensation des gasförmigen NH₃ kann entweder direkt bei seinem Einleiten in das flüssige NH₃ erfolgen, oder aber bevorzugt durch Kondensation des laufend sich bildenden gereinigten NH₃, beispielsweise mit Hilfe eines nachgeschalteten Kühlers.

Beim Durchleiten des zu reinigenden NH₃ durch das flüssige NH₃ entsteht mit Melamin angereichertes flüssiges NH₃, das je nach Druck und Temperatur mehr oder minder große Mengen Melamin enthält. Es kann mit Melamin gesättigt oder untersättigt sein, es kann sich auch bereits festes Melamin gebildet haben, das heißt, unter melaminhältigem flüssigem NH₃ ist sowohl eine Lösung als auch eine Suspension von Melamin in NH₃ zu verstehen.

In einem kontinuierlichen Verfahren, in dem das mit Melamin angereicherte NH₃ laufend etwa über einen Überlauf ausgeschleust wird, wird das mit Melamin angereicherte flüssige NH₃ üblicherweise in einen geeigneten Abschnitt des Melaminherstellungsverfahrens rückgeführt. Dies kann der Reaktor oder ein anschließender Separator sein. Vorteilhaft ist die Rückführung in eine Einrichtung, in der flüssiges Melamin unter Ammoniakdruck verweilen gelassen wird ("Aging"), oder in eine Einrichtung, in der bereits verfestigtes Melamin unter Ammoniakdruck verweilen gelassen wird ("Tempern").
Bevorzugt wird das melaminhältige flüssige NH₃ in eine Anlage rückgeführt, in der flüssiges Melamin unter Entspannung mit Hilfe von flüssigem Ammoniak oder des beim Entspannen des flüssigen Ammoniaks gebildeten gasförmigen Ammoniak gequencht (verfestigt) wird. Ebenso bevorzugt ist es, das melaminhältige flüssige NH₃ in eine Anlage rückzuführen, in der eine Mischung von gasförmigem NH₃ und gasförmigem Melamin mit Hilfe von Ammoniak gequencht und verfestigt wird.

In einer weiteren, bevorzugten Ausführungsform wird das melaminhältige flüssige NH₃ in ein aus festem Melamin oder festem Melamin und festen Inertstoffen bestehendes und mit NH₃-Gas aufrecht erhaltenes Wirbelbett rückgeführt.

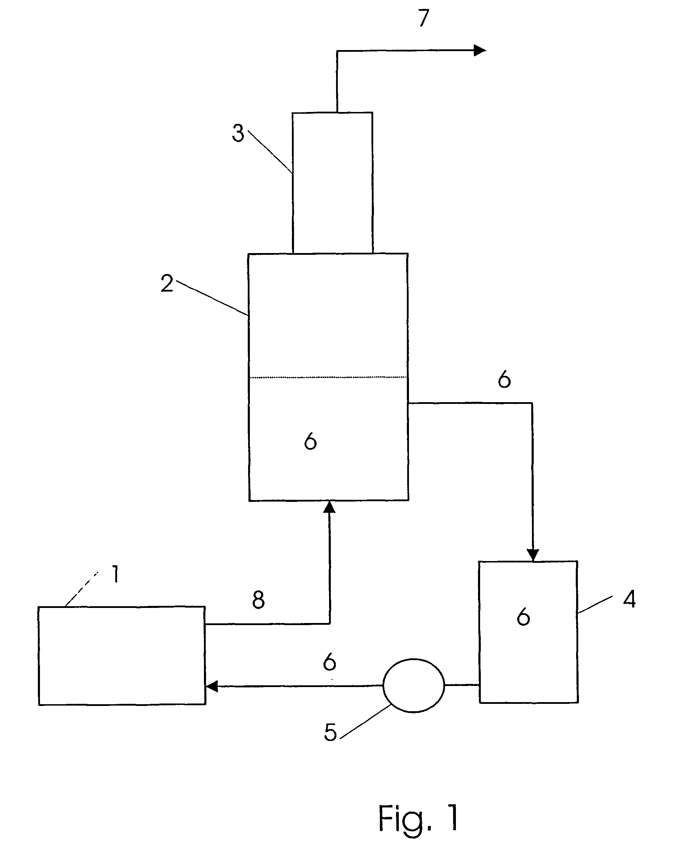

Eine mögliche Anlage zur Durchführung des erfindungsgemäßen Verfahrens ist schematisch in Fig. 1 dargestellt. Darin bedeuten:
(1) Wirbelbett
(2) Druckgefäß
(3) Kühler
(4) Zwischenbehälter
(5) Pumpe
(6) mit Melamin angereichertes flüssiges NH₃
(7) gasförmiges, melaminfreies NH₃
(8) gasförmiges melaminhältiges NH₃

### Beispiel 1:

Ein aus dem Wirbelbett einer Melamin-Verfestigungsanlage kommender, melaminhältiger gasförmiger NH₃-Strom von 424 kg/h wird bei einer Temperatur von 280 °C und einem Druck von 10 bar in einem Druckgefäß mit Kühler, das mit flüssigem NH₃ gefüllt ist, von unten durch das flüssige NH₃ geleitet. Dabei kondensiert ein Teil des NH₃, und das Melamin scheidet sich im flüssigen NH₃ ab. Zur Aufrechterhaltung eines konstanten Flüssigkeitsstandes im Druckgefäß werden 384 kg/h flüssiges, melaminhältiges NH₃ ausgeschleust und in die Melaminanlage rückgeführt. Das im Druckgefäß verdampfende, gereinigte NH₃-Gas kondensiert teilweise im aufgesetzten Kühler, der nicht kondensierte Anteil wird als Rein-NH₃-Gas in einer Menge von 40 kg/h abgezogen.

### Beispiel 2-4:

Analog zu Beispiel 1 wurde melaminhältiges NH₃ Gas bei verschiedenen Drücken und Temperaturen gereinigt. Die entsprechenden Werte sind in Tabelle 1 aufgelistet.

**Tabelle 1:**

| Beispiel | Temp. (°C) | Druck (bar) | zu reinigendes NH₃ (kg/h) | rückgeführtes NH₃ (kg/h) | reines NH₃ (kg/h) |
|---|---|---|---|---|---|
| 1 | 280 | 10 | 424 | 384 | 40 |
| 2 | 280 | 40 | 740 | 650 | 87 |
| 3 | 340 | 10 | 380 | 340 | 40 |

## Patentansprüche

1. Verfahren zur Reinigung von melaminhältigem, gasförmigem NH₃, **dadurch gekennzeichnet, daß** es durch Abkühlen teilweise kondensiert wird, wobei sich das Melamin im flüssigen NH₃ anreichert und das gereinigte NH₃-Gas abgezogen wird.

2. Verfahren nach Anspruch 1, **dadurch gekennzeichnet, daß**
- das melaminhältige, gasförmige NH₃ durch flüssiges NH₃ geleitet, dabei abgekühlt und teilweise kondensiert wird,
- das gereinigte NH₃-Gas abgezogen und
- mit Melamin angereichertes flüssiges NH₃ ausgeschleust wird.

3. Verfahren nach Anspruch 2, **dadurch gekennzeichnet, daß** das mit Melamin angereicherte flüssige NH₃ das Melamin gelöst enthält.

4. Verfahren nach Anspruch 2, **dadurch gekennzeichnet, daß** das mit Melamin angereicherte flüssige NH₃ festes Melamin enthält.

5. Verfahren nach Anspruch 2, **dadurch gekennzeichnet, daß** das mit Melamin angereicherte flüssige NH₃ in eine Anlage zum Verfestigen von flüssigem oder gasförmigem Melamin rückgeführt wird.

6. Verfahren nach Anspruch 2, **dadurch gekennzeichnet, daß** das mit Melamin angereicherte flüssige NH₃ in ein aus festem Melamin oder festem Melamin und festen Inertstoffen bestehendes und mit NH₃-Gas aufrecht erhaltenes Wirbelbett rückgeführt wird.

7. Verfahren nach Anspruch 1, **dadurch gekennzeichnet, daß** das melaminhältige NH₃-Gas aus einem Verfahren zum Verfestigen von flüssigem, unter NH₃-Druck stehendem Melamin mit Hilfe von flüssigem, überkritischem oder gasförmigem Ammoniak stammt.

8. Verfahren nach Anspruch 1, **dadurch gekennzeichnet, daß** das melaminhältige NH₃-Gas aus einem Verfahren zum Verfestigen von gasförmigem, unter NH₃-Druck stehendem Melamin mit Hilfe von flüssigem, überkritischem oder gasförmigem NH₃ stammt.

9. Verfahren nach Anspruch 1, **dadurch gekennzeichnet, daß** das melaminhältige NH₃-Gas aus dem Wirbelbett einer Anlage zur Verfestigung von flüssigem Melamin stammt.

10. Verfahren nach Anspruch 1, **dadurch gekennzeichnet, daß** das melaminhältige NH₃-Gas aus einem Wirbelbett einer Anlage zur Verfestigung von gasförmigem Melamin stammt.

## Claims

1. Process for purifying melamine-containing gaseous NH₃, **characterized in that** said melamine-containing gaseous NH₃ is partly condensed by cooling, the melamine accumulating in the liquid NH₃ and the purified NH₃ gas being taken off.

2. Process according to Claim 1, **characterized in that**
- the melamine-containing gaseous NH₃ is passed through liquid NH₃, being cooled and partly condensed,
- the purified NH₃ gas is taken off and
- melamine-enriched liquid NH₃ is discharged.

3. Process according to Claim 2, **characterized in that** the melamine-enriched liquid NH₃ contains the melamine in dissolved form.

4. Process according to Claim 2, **characterized in that** the melamine-enriched liquid NH₃ contains solid melamine.

5. Process according to Claim 2, **characterized in that** the melamine-enriched liquid NH₃ is recirculated to a plant for solidifying liquid or gaseous melamine.

6. Process according to Claim 2, **characterized in that** the melamine-enriched liquid NH₃ is recirculated to a fluidized bed which consists of solid melamine, or solid melamine and solid inert matter, and is maintained using NH₃ gas.

7. Process according to Claim 1, **characterized in that** the melamine-containing NH₃ gas originates from a process for solidifying liquid melamine under NH₃ pressure using liquid, supercritical or gaseous ammonia.

8. Process according to Claim 1, **characterized in that** the melamine-containing NH₃ gas originates from a process for solidifying gaseous melamine under NH₃ pressure using liquid, supercritical or gaseous NH₃.

9. Process according to Claim 1, **characterized in that** the melamine-containing NH₃ gas originates from the fluidized bed of a plant for solidifying liquid melamine.

10. Process according to Claim 1, **characterized in that** the melamine-containing NH₃ gas originates from a fluidized bed of a plant for solidifying gaseous melamine.

## Revendications

1. Procédé d'épuration de NH₃ gazeux contenant de la mélamine, **caractérisé en ce que** le NH₃ est condensé par refroidissement et **en ce que** la mélamine se concentre dans le NH₃ liquide, le NH₃ gazeux épuré étant extrait.

2. Procédé selon la revendication 1, **caractérisé en ce que** :
- le NH₃ gazeux qui contient de la mélamine est passé à travers du NH₃ liquide en étant ainsi refroidi et partiellement condensé,
- **en ce que** le NH₃ gazeux épuré est extrait et
- **en ce que** le NH₃ liquide enrichi en mélamine est séparé.

3. Procédé selon la revendication 2, **caractérisé en ce que** le NH₃ liquide enrichi en mélamine contient la mélamine à l'état dissous.

4. Procédé selon la revendication 2, **caractérisé en ce que** le NH₃ liquide enrichi en mélamine contient de la mélamine solide.

5. Procédé selon la revendication 2, **caractérisé en ce que** le NH₃ liquide enrichi en mélamine est renvoyé dans une installation de solidification de la mélamine liquide ou gazeuse.

6. Procédé selon la revendication 2, **caractérisé en ce que** le NH₃ liquide enrichi en mélamine est renvoyé dans un lit fluidisé constitué de mélamine solide ou de mélamine solide et de substances inertes solides et maintenu à l'aide de NH₃ gazeux.

7. Procédé selon la revendication 1, **caractérisé en ce que** le NH₃ gazeux qui contient de la mélamine provient d'un procédé de solidification de mélamine liquide sous pression de NH₃, à l'aide d'ammoniac liquide, hypercritique ou gazeux.

8. Procédé selon la revendication 1, **caractérisé en ce que** le NH₃ gazeux qui contient de la mélamine provient d'un procédé de solidification de mélamine gazeuse placée sous pression de NH₃, à l'aide de NH₃ liquide, hypercritique ou gazeux.

9. Procédé selon la revendication 1, **caractérisé en ce que** le NH₃ gazeux qui contient de la mélamine provient du lit fluidisé d'une installation de solidification de mélamine liquide.

10. Procédé selon la revendication 1, **caractérisé en ce que** le NH₃ gazeux qui contient de la mélamine provient d'un lit fluidisé d'une installation de solidification de mélamine gazeuse.
